# EUROPEAN PATENT APPLICATION

(11) **EP 3 679 927 A1**
(43) Date of publication of application: **15.07.2020**
(21) Application number: 19151604.6
(22) Date of filing: 14.01.2019
(51) Int. Cl.: A61K 9/20, A61K 9/48, A61K 47/12, A61K 31/167, A61K 31/395

(54) **A METHOD OF MANUFACTURING A PHARMACEUTICAL COMPOSITION COMPRISING NEFOPAM AND ACETAMINOPHEN, AND THE PHARMACEUTICAL COMPOSITION OBTAINED THEREBY**

(71) Applicant: APTYS Pharmaceuticals SAS, 63360 Saint Beauzire (FR)
(72) Inventor: BOUTIGNON, François, 63000 Clermont-Ferrand (FR); BERNARD, Wahéna, 63200 Riom (FR); MASSON-SINTEFF, Claudie, 63200 Mosac (FR)
(74) Representative: Holme Patent A/S

(57) **Abstract**

The present invention relates to a method of preparing a pharmaceutical composition comprising acetaminophen and nefopam, and wherein the wherein the pH-value of the pharmaceutical composition is adjusted to be between 4.8 and 5.8, preferably around 5.2. When the pH-value of the pharmaceutical composition is adjusted to be between 4.8 and 5.8, a stable pharmaceutical composition is obtained which is substantial free from impurities, i.e. the degradation impurities from nefopam and acetaminophen is below the Limit of Detection (LOD) at accelerated aging tests.

## Description

The present invention relates to a pharmaceutical composition comprising the two active ingredients nefopam and acetaminophen, a method of manufacturing said pharmaceutical composition, and the use of said composition.

The literature indicates that the control of pain, e.g. postoperative pain, remains a challenge for several reasons, including delayed patient recovery and the risk of developing persistent postoperative pain.

Opioids, non-steroidal anti-inflammatory drugs (NSAIDs) and acetaminophen are widely used for the treatment of moderate to severe pain, and they are today the most commonly used analgesics for the management of chronic, inflammatory and postoperative pain.

These agents consistently produce analgesia, but they have a number of undesirable side effects that limit their clinical usefulness. The side effects of opioids include nausea, vomiting, constipation, respiratory depression, urinary retention, sedation, tolerance and physical dependence, while gastrointestinal troubles are frequently observed with NSAIDs, and liver injury is associated with acetaminophen.

This situation has led to the co-administration of combinations of analgesics that have different mechanisms of action through a strategy called 'multimodal' or 'balanced' analgesia.

The mechanism underlying the use of multimodal analgesia is that to use analgesics having a different mode of analgesic action, which allows the dose of the analgesics to be reduced and results in a lowered incidence of side effects. The basic goal of this strategy is a synergistic, or at least, additive, analgesic interaction between the combined drugs. However, there is no consensus regarding what the ideal approach to multimodal analgesia is.

Several different multimodal approaches have been suggested in the literature, most of which includes the use of opioids. However, due to the sever side effect of opioids, is may in some situations be preferred to use non-opioids, and here nefopam has proven to be beneficial (Girard et al., Systematic evaluation of the nefopam-paracetamol combination in rodent models of antinociception; 2011)

Nefopam (5-methyl-1-phenyl-1,3,4,6-tetrahydro-2,5-benzoxazocine) is an analgesic that has been used to treat mild to moderate postoperative pain in different clinical settings. It is a non-opioid and non-steroidal drug, that is chemically distinct and pharmacologically unrelated to any of the presently known analgesics. (Heel et al., Nefopam: A review of its pharmacological properties and therapeutic efficacy. Drugs 1980; 19: 249-67).

Furthermore, the use of nefopam is advantageously since it does not have any of the known side-effects normally associated with opioids, that is, it does not bind to opioid receptors, does not cause respiratory depression, has no effect on platelet function and does not induce an anti-inflammatory effect.

Its main mechanisms of analgesic action involve the inhibition of serotonin, norepinephrine and dopamine reuptake, and effects on the glutamatergic pathway via modulations of calcium and sodium channels that leads to decreased activation of postsynaptic glutamatergic receptors, such as *N*-methyl-D-aspartate (NMDA) receptors, which are involved in the development of hyperalgesia.

However, the short elimination half-life of nefopam (four hours) makes it difficult to maintain analgesic efficacy over the normal dosing period (three times daily). Dose escalation of nefopam brings about an increase in the frequency of adverse drug reactions associated with the analgesic, and adverse effects on pulse and blood pressure have been observed following parenteral delivery of therapeutic doses of nefopam.

Accordingly nefopam, is a good candidate for inclusion in multimodal analgesia. One of the compounds nefopam has been combined with if order to obtain a multimodal analgesia, is acetaminophen (N-acetyl-*p*-amino-phenol), also known as paracetamol. Acetaminophen is a widespread antipyretic and analgesic, and is accordingly accepted as an effective treatment for the relief of pain and fever in both adults and children.

In preparation and storage of pharmaceutical compositions it is important to provide the active drugs in a pure form. Moreover, it is desirable to achieve this high purity and stability with as simple a formulation as possible. However, one of the problems with pharmaceutical compositions is that impurities may appear in the compositions, e.g. due to choice of synthetic route, the quality of the starting materials, the reaction conditions, the final purification step, the design of the process equipment etc. The presence of such unwanted chemicals, even in trace amount, may influence the efficacy and safety of the pharmaceutical composition.

One of the main impurity problems in relation to acetaminophen, originates as a consequence of both synthesis and degradation of acetaminophen during storage, where acetaminophen is converted into 4-aminophenol.

Even though acetaminophen when stored at room temperature (about 20°C) under dry conditions is considered to be stable, the compound will at elevated temperatures, e.g. in tropical countries, and in the presence of trace moisture degrade rapidly to 4-aminophenol, which subsequently undergoes additional oxidative changes and be converted into p-benzoquinone and hydroquinone, both of which decomposes rapidly at room temperature. (Fairbrother, J.E. "Acetominophen". In Analytical Profiles of Drug Substances, Vol. 3. K. Florey, Ed. Academic Press, New York, NY, 1974, pp. 1-110.).

Furthermore, in aqueous solutions the degradation of acetaminophen is both acid and base catalyzed and degrades via first order kinetics to 4-aminophenol (Koshy et al., Stability of aqueous solutions of N-acetylp-aminophenol. J. Pharm. Sci. 50: 113-18 (1961)).

Since 4-aminophenol is reported to have both nephrotoxic and teratogenic effects (Németh et al., Determination of paracetamol and its main impurity 4-aminophenol in analgesic preparations by micellar electrokinetic chromatography, Journal of Pharmaceutical and Biomedical Analysis, 2008, vol. 47 (pg. 746-749)), the quantity of 4-aminophenol in the pharmaceutical composition must be strictly controlled.

In a similar manner, undesirable degradation products (impurities) from nefopam can be found in products containing nefopam, and there is accordingly a demand to provide simple formulations and processes for preparation of pharmaceutical compositions containing acetaminophen and nefopam which have low levels of impurities.

Thus, it is a first aspect according to the present invention to provide a stable pharmaceutical composition comprising nefopam and acetaminophen that may be stored in a humid environment and/or at elevated temperatures without the paracetamol degrades to 4-aminophenol.

It is a second aspect according to the present invention to provide a stable pharmaceutical composition comprising nefopam and acetaminophen that has undetectable levels of impurities irrespectively of the storage conditions.

It is a third aspect according to the present invention to provide a new manufacturing process arranged for providing a stable pharmaceutical composition.

It is a fourth aspect according to the present invention to provide a new manufacturing process which is operationally simple, easy to handle and applicable at an industrial scale.

These and further aspects are achieved according to the present invention by providing a method of preparing a pharmaceutical composition comprising acetaminophen and nefopam, said method comprises
a. in a first process step providing a granulated powder by mixing acetaminophen, nefopam and one or more excipients;
b. in a second process step mixing a lubricant with the granulated powder,
c. in a third process step, forming the pharmaceutical composition, and
wherein the pH-value of the pharmaceutical composition is adjusted to be between 4.8 and 5.8, preferably around 5.2.

Acetaminophen is stabile in a basic environment and nefopam is stable in acidic environment, and the inventor of the present invention has found, that if the pH-value of the pharmaceutical composition is adjusted to be between 4.8 and 5.8, preferably around 5.2, the method according to the invention will provide a stable pharmaceutical composition which is substantial free from impurities, i.e. the degradation impurities from nefopam and acetaminophen is below the Limit of Detection (LOD) at accelerated aging tests. Said pH-value may be adjusted in the first and/or second process step by using one or more pH adjusting agent(s).

Said pH adjusting agent(s) may be any suitable compound capable of adjusting the pH-value in final pharmaceutical composition, however in a preferred embodiment the pH-value in the first process step is adjusted using citric acid, and/or the pH-value in the second process step is adjusted using magnesium stearate.

The inventors have found that by using the above method, the provided pharmaceutical composition will be more stable, such that said composition will comprise fewer impurities than using the known preparation methods for multimodal analgesia comprising acetaminophen and nefopam. Thus, the inventors of the present invention have found surprising effects on the stability of the acetaminophen and nefopam in the pharmaceutical composition using the above method.

In the present application the term "impurities" are defined as "substances in the pharmaceutical composition that are not the active pharmaceutical ingredients (API) themselves, i.e. acetaminophen and nefopam, or the excipients used to manufacture the composition", i.e. impurities are unwanted chemicals that remain within the formulation in small amounts and which may influence quality, safety and efficacy of the composition, thereby potentially causing serious health hazards.

The inventors of the present invention have found that the concentration of impurities in the pharmaceutical composition is well below the thresholds specified by e.g. the European, United States, British and German Pharmacopoeias, e.g. employing a conventional HPLC method, which is set to be 1000 ppm or 0.1% w/w.

Analysis of impurities (including degradants) is done using reverse phase HPLC techniques on the respective samples as is known in the art. Calculations of the amount of impurities is expressed as the integrated area percent of the impurity peak(s) divided by the integrated area percent of all drug-related peaks.

The inventors of the present invention have found that a preferred ratio between nefopam and acetaminophen in the pharmaceutical composition is between 1/10 and 1/30, preferably between 1/15 and 1/20.

In a preferred embodiment the first process step comprises the following consecutive steps:
- adding water to the granulated powder obtained in step a) thereby providing a first mixture; and
- drying the first mixture to a water content of between 2 and 5 wt%.

In this way the granulation step, becomes a wet granulation step, and the water mixed into the powders will likely form bonds between the respective powder particles thereby locking the particles together.

Once the solvent/water has been dried and the powders have formed a more densely mass contain small amounts of water, then the granulation may optionally be milled/ mixed, homogenised etc.

In the first process step, a number of excipients suitable for use in a granulation step are added to the acetaminophen, and nefopam. Said excipients are preferably selected from the group consisting of granulating agents, diluents, solvents, glidants, surfactants, preservatives, solubilizers, emulsifiers, plasticizers and the like. The number of excipients that can be included in a formulation is not limited.

Examples of diluents/fillers include, but not limited to, celluloses, cellulose acetate, microcrystalline cellulose, co-processed microcrystalline celluloses (such as various grades of Avicel), silicified microcrystalline cellulose, dextrates, dextrin, dextrose, fructose, glyceryl palmitostearate, hydrogenated vegetable oil, kaolin, lactitol, lactose, maltitol, mannitol, maltodextrin, maltose, pregelatinized starch, sodium chloride, sorbitol, starches, sucrose, glucose, trehalose, erythritol, fructose, calcium sulphate, dibasic calcium phosphate, talc and xylitol or a mixture of one or more of said diluents. However, in a preferred embodiment the diluents/filler is microcrystalline cellulose.

Suitable binders include, but not limited to, celluloses such as microcrystalline cellulose, modified celluloses such as low substituted hydroxypropyl cellulose, hydroxypropyl cellulose, hydroxypropyl methylcellulose, hydroxyethylcellulose, hydroxyethyl methylcellulose, cellulose gum, xanthan gum, sugars (such as sucrose, glucose, amylose, maltodextrin, dextrose and the like), starches such as corn or potato starch, pregelatinized starches, polyvinyl alcohol- polyethylene glycol graft copolymer, copovidone, povidone, carbomers, polycarbophil, polyethylene oxide, polyethylene glycol or a combination of suitable binders. However, in a preferred embodiment the binder is povidone K90 and/or Starch.

Examples of disintegrants include, but not limited to starches, partially pregelatinized starches, sodium starch glycolate, pregelatinized starch, alginic acid, powdered cellulose, croscarmellose sodium, crospovidone, docusate sodium, guar gum, hydroxypropyl cellulose, low substituted hydroxypropyl cellulose, magnesium aluminum silicate, methylcellulose, sodium alginate or a combination of one or more disintegrants. However, in a preferred embodiment the disintegrant is croscarmellose sodium.

In the second process step, a lubricant is added. Examples of lubricants include, but is not limited to, calcium stearate, zinc stearate, magnesium stearate, aluminium stearate, stearic acids, sodium stearyl fumarate, hydrogenated castor oil, light mineral oil, polyethylene glycol, magnesium lauryl sulfate and the like. However, in a preferred embodiment the lubricant is magnesium stearate.

In a preferred embodiment the first process step is further divided into the consecutive steps of:
a' adding a part of the acetaminophen to a first mixing vessel,
a" adding the one or more excipientsto the first mixing vessel,
a"' adding the remaining acetaminophen to the first mixing vessel, and mixing to a homogenous powder.

By layering the different components in the mixing vessel in it is ensured that the different components, especially acetaminophen, does not clump together. That is the one or more excipients are also functioning as suspending agent(s), i.e. they aid in providing a homogeneous composition during mixing. Without being bound by theory it is believed that the individual excipients ensures that the acetaminophen particles are separated and accordingly evenly distributed in the dry mixture.

The ratio of the acetaminophen to the one or more excipients in the dry mixture initially obtained in the first process step is preferably about 2:1 to 5:1, more preferably 2.4:1 to 2.8:1 as the inventors have shown said ratios provides the best results.

In order to obtain a suitable dry mixture for the further process steps in the method according to the invention, it is preferred that the compounds after addition to the first mixing vessel is mixed until the dry mixture is homogenous. The mixing conditions will vary depending on the first mixing vessel and the quantity.

In order to ensure an even distribution of acetaminophen in the dry mixture, as well as reducing the mixing time required for providing a homogeneous dry mixture, it is preferred that half of the acetaminophen is added to the first mixing vessel in step a' and the remaining in step a"'.

In a similar manner the second process step may be divided into the following consecutive steps:
b' adding a part of the granulated powder obtained in the first process step to a second mixture vessel,
b" adding the lubricant and the nefopam to the second mixing vessel,
b"' adding the remaining of the granulated powder obtained in the first process step to the second mixing vessel, and mixing.

In order to obtain a homogeneous mixture it is preferred that half of granulated powder obtained in the first process step is added to the second mixing vessel in step b'.

The present invention also relates to a pharmaceutical composition, preferably obtained by the method according to the present invention. Said composition comprises acetaminophen and nefopam, and wherein the pH-value of the pharmaceutical composition is between 4.8 and 5.8, preferably around 5.2.

Said pharmaceutical composition will after storage at 40°C and 75% relative humidity for 4 weeks, preferably contain no more than about 0.05% total impurities based on area percent of drug related HPLC peaks.

In a preferred embodiment the content of 4-aminophenol, i.e. the main degradation product of acetaminophen, after storage at 40°C and 75% relative humidity for 4 weeks, is less than 0.001% based on area percent of drug related HPLC peaks. Accordingly, this limit is well below the 50 ppm or 0.005% w/w threshold specified by the European, United States, British and German Pharmacopoeias

In a similar way the content of impurities originating from nefopam in the pharmaceutical composition after storage at 40°C and 75% relative humidity for 4 weeks, is less than 0.05% based on area percent of drug related HPLC peaks.

In a preferred embodiment the ratio between nefopam and acetaminophen in the pharmaceutical composition is between 1/10 and 1/30, preferably between 1/15 and 1/20.

The invention also relates to a liquid or a solid unit dosage of the pharmaceutical composition according to the invention. Said solid unit dosage may be a tablet or a capsule arranged for oral administration, but other administration forms are also contemplated within the scope of the present invention.

In a preferred embodiment the unit dosage comprises between 5 and 100 mg nefopam, preferably between 10 and 50 mg nefopam and even more preferred between 20 and 40 mg nefopam.

The unit dosage furthermore comprises between 100 and 1000 mg acetaminophen, preferably between 200 and 750 mg acetaminophen and even more preferred between 300 and 600 mg acetaminophen.

In this respect the median effective analgesic dose (median value and 95% confidence interval) of nefopam and acetaminophen were 30 mg and 500 mg, respectively. Thus, a preferred oral unit dosage for management of pain, having a pH-value around 5.2 comprises the following components

| Components | (mg) |
|---|---|
| Acetaminophen | 500 |
| Microcrystalline Cellulose | ≈146.7 |
| Povidone K90 | ≈33,0 |
| Starch | ≈18,3 |
| Anhydrous citric acid | ≈1,5 |
| Nefopam HCL | 30,0 |
| Magnesium stearate | ≈4,0 |

### EXAMPLES

### EFFECT OF pH ON STABILITY OF NEFOPAM AND ACETAMINOPHEN

In order to evaluate if the pH-value had an effect on the stability of the nefopam and/or acetaminophen, a number of pharmaceutical compositions (batches) were prepared using the method according to the present invention. The stability of batches differing in pH-value were compared.

In the context of the present invention the term % refers to percentage by weight of the composition.

The following four batches were prepared as follows:

### Batch F232H094

### First process step

A granulated powder was prepared in a first process step, by adding the following to a planetary mixer: acetaminophen (API), microcrystalline cellulose (a diluent), Povidone K90 and Starch (binders), Croscarmellose sodium (a disintegrant) and Anhydrous citric acid (a pH adjusting agent).

The components were added to a planetary mixer in the following order: Half of acetaminophen, microcrystalline cellulose, starch, PVP K90, cross-carmellose sodium, anhydrous citric acid, and finally the second half of acetaminophen.

The resultant initial mixture, 800 g, was mixed for 10 min at 105 rpm.

The composition of the initial mixture was the following:

| | |
|---|---|
| Acetaminophen | 68.16% |
| Microcrystalline cellulose | 15.85% |
| Povidone K90 | 6.00% |
| Starch | 4.95% |
| Croscarmellose sodium | 0.35% |
| Anhydrous citric acid | 0.40% |

Purified water (256 g) was added into the planetary mixer containing the initial mixture, under constant stirring at 105 rmp for 5 min. Stirring was thereafter continued for 10 min at 130 rpm.

The resulting powder was spread on four plates (200 g/plate), the plates were heated at 50°C until the water content reached 2.7 to 3.5 wt%. The resulting product was cooled down for 15 min and granulated using a wet granulator with a 1.25 mm grid with a 40 rpm speed. The final granulated powder (was homogenised using a Turbula mixer for 5 min at 51 rpm.

### Second process step

The external phase used was as follows:

| | |
|---|---|
| Nefopam HCl | 4.09% |
| Magnesium stearate | 0.2% |

Half of the granulated powder from the first process step was poured in a 1L flask and the external phase was added followed by the second half of the granulated powder. The composition was then mixed using a Turbula mixer (15 min, 23 rpm).

The final powder (350 g) was then tabletted.

### F238H100

### First process step

The first process step were performed as described for batch F232H094.

The composition of the initial mixture was the following:

| | |
|---|---|
| Acetaminophen | 68.16% |
| Microcrystalline cellulose | 17.55% |
| Povidone K90 | 4.50% |
| Starch | 4.95% |
| Croscarmellose sodium | 0.35% |
| Anhydrous citric acid | 0.20% |

Purified water (256 g) was added into the planetary mixer under constant stirring at 105 rmp for 5 min. Stirring was continued for 10 min at 130 rpm.

The drying and homogenisation steps were performed as described for batch F232H094.

### Second process step

The external phase used was as follows:

| | |
|---|---|
| Nefopam HCl | 4.09% |
| Magnesium stearate | 0.2% |

Half of the granulated powder was poured in a 1L flask and the external phase was added followed by the second half of the granulated powder. The composition was then mixed using a Turbula mixer (15 min, 73 rpm).

The final powder (350 g) was then tabletted.

### F248H121

### First process step

The first process step were performed as described for batch F232H094.

The composition of the initial mixture was the following:

| | |
|---|---|
| Acetaminophen | 68.16% |
| Microcrystalline cellulose | 17.20% |
| Povidone K90 | 4.50% |
| Starch | 4.95% |
| Croscarmellose sodium | 0.35% |
| Anhydrous citric acid | 0.20% |

Purified water (236 g) was added into the planetary mixer containing the initial mixture under constant stirring at 105 rmp for 10 min. Stirring was continued for 10 min at 130 rpm.

The drying and homogenisation steps were performed as described for batch F232H094.

### Second process step

The external phase used was as follows:

| | |
|---|---|
| Nefopam HCl | 4.09% |
| Magnesium stearate | 0.55% |

Half of the granulated powder was poured in a 1L flask and the external phase was added followed by the second half of the granulated powder. The composition was then mixed using a Turbula mixer (5 min, 51 rpm).

The final powder (350 g) was then tabletted.

### F236H098

### First process step

The first process step were performed as described for batch F232H094.

The composition of the initial mixture was the following:

| | |
|---|---|
| Acetaminophen | 68.16% |
| Microcrystalline cellulose | 15.25% |
| Povidone K90 | 6.00% |
| Starch | 4.95% |
| Croscarmellose sodium | 0.35% |
| Anhydrous citric acid | 0.20% |

Purified water (236 g) was added into the planetary mixer containing the initial mixture under constant stirring at 105 rmp for 5 min. Stirring was done for 10 min at 130 rpm.

The drying and homogenisation steps were performed as described for batch F232H094.

### Second process step

The external phase used was as follows:

| | |
|---|---|
| Nefopam HCl | 4.09% |
| Magnesium stearate | 1.00% |

Half of the granulated powder was poured in a 1L flask and the external phase was added followed by the second half of the granulated powder. The composition was then mixed using a Turbula mixer (15 min, 23 rpm).

The final powder (350 g) was then tabletted.

### pH-value in the batches F232H094, F238H100, F248H121 and F236H098

The measure of pH was carried out as follows:
2 tablets was dissolved in 100 g of water, and heated to a temperature of 40°C (about 15 min) under magnetic stirring, and then maintained at a temperature of 40°C for 30 min under magnetic stirring.

The shambles were cool down at room temperature.
The pH-value were measured using a conventional pH-meter after 10 min of magnetic stirring.

The pH of the four batches were:

| **Batch numbers:** | **F232H094** | **F238H100** | **F248H121** | **F236H098** |
|---|---|---|---|---|
| pH | 3.98 | 4.57 | 5.24 | 6.28 |

### Stability test - comparing batches F232H094, F238H100, F248H121 F236H098

The tablets were packaged in glass bottles and then placed in stability chambers at 40°C/75%RH.

Samples were taken after two and four weeks and the level of degradation product was assayed using two HPLC methods.

### For nefopam:

Kinetex C18 100A column; mobile phase, A: KH₂PO₄-phosphoric acid buffer, B: Acetonitrile; gradient, 75% A to 30%A for 10 min; flow rate 1.5 mL/min; oven temperature 30°C; detection 210 nm; volume of injection 10 pL.

The impurities of nefopam were essayed using external standards. Three unknown impurities were detected at 0.068, 1.12, and 1.17 relative retention time (RR).

The LOD of the method is 0.05% and the LOQ was 0.0155%.

### For acetaminophen:

Zorbax RX C8 column; mobile phase, A: Na₂HPO₄, NaH₂PO₄, B: Methanol R containing tetrabutylammonium hydroxide; gradient, 75% A to 55%A for 33 min; flow rate 1.5 mL/min; oven temperature 45°C; detection 245 nm; volume of injection 20 pL.

The impurities of acetaminophen were essayed using external standards. Impurities were detected at 0.73 (4-Aminophenol, Imp K), 3.0 (4-Nitrophenol, Imp F), 6.3 (4-Chloroacetanalide, Imp J), 7.1 (2-Benzoybenzoic acide, Imp Nefopam) and 8.3 (Nefamide, Imp Nefopam) relative retention time (RR).

The LOD of the method is 0.00025% and the LOQ was 0.00075%. The results of impurities levels are presented in the following table:

**ND: not detected**

| **Batch N°** | **pH** | **2 weeks at 40°C/75%RH Acetaminophen main impurity (4 amino phenol)** | **4 weeks at 40°C/75%RH Acetaminophen main impurity (4 amino phenol)** | **2 weeks at 40°C/75%RH Nefopam main impurity** | **4 weeks at 40°C/75%RH Nefopam main impurity** |
|---|---|---|---|---|---|
| F232H094 | 3.98 | 0.0038 | 0.0062 | ND | ND |
| F238H100 | 4.57 | 0.0010 | 0.0016 | ND | ND |
| F248H121 | 5.24 | <LOQ | 0.00096 | ND | ND |
| F236H098 | 6.28 | ND | <LOQ | <LOQ | <LOQ |

As acetaminophen preferably is stable in basic environment and nefopam is stable in acidic environment, the inventors of the present invention found that a pH about 5.2 was the best pH were both compounds were stable. This pH was obtained according to the amount of citric acid and magnesium stearate.

## Claims

1. A method of preparing a pharmaceutical composition comprising acetaminophen and nefopam, said method comprises
a. in a first process step providing a granulated powder by mixing acetaminophen, nefopam and one or more excipients;
b. in a second process step mixing a lubricant with the granulated powder,
c. in a third process step, forming the pharmaceutical composition, and
wherein the pH value of the pharmaceutical composition is adjusted to between 4.8 and 5.8, preferably around 5.2.

2. The method according to claim 1, wherein the first process step further comprises the following consecutive steps:
- adding water to the granulated powder obtain in step a) thereby providing a first mixture; and
- drying the first mixture to a water content of between 2 and 5 wt%.

3. The method according to claim 1 or 2, wherein step a) of the first process step is divided into the consecutive steps:
a' adding a part of the acetaminophen to a first mixing vessel,
a" adding the one or more of the excipientsto the first mixing vessel,
a'" adding the remaining acetaminophen to the first mixing vessel, and mixing.

4. The method according to claim 3, wherein about half of the acetaminophen is added to the first mixing vessel in step a' .

5. The method according to any of the preceding claims, wherein step b) of the second process step is divided into the following consecutive steps:
b' adding a part of the granulated powder obtained in the first process step to a second mixture vessel,
b" adding the lubricant and to the second mixing vessel,
b"' adding the remaining of the granulated powder obtained in the first process step to the second mixing vessel, and mixing.

6. The method according to claim 5, wherein about half of the granulated powder obtain in the first process step is added to the second mixing vessel in step b'.

7. A pharmaceutical composition obtained according to any of the claims 1 - 6, comprising the active ingredients acetaminophen and nefopam, and wherein the pH value in the pharmaceutical composition is between 4.8 and 5.8, preferably around 5.2.

8. The pharmaceutical composition according to claim 7, wherein the pharmaceutical composition after storage at 40°C and 75% relative humidity for 4 weeks, contains not more than about 0.05% total impurities based on area percent of drug related HPLC peaks, and wherein

9. The pharmaceutical composition according to claim 8, wherein the content of 4-aminophenol in the pharmaceutical composition after storage at 40°C and 75% relative humidity for 4 weeks, is less than 0.001% based on area percent of drug related HPLC peaks.

10. The pharmaceutical composition according to claim 7, 8 or 9, wherein the content of impurities originating from nefopam in the pharmaceutical composition after storage at 40°C and 75% relative humidity for 4 weeks, is less than 0.05% based on area percent of drug related HPLC peaks.

11. The pharmaceutical composition according to any of the claims 7 - 10, wherein the ratio between nefopam and acetaminophen in the pharmaceutical composition is between 1/10 and 1/30, preferably between 1/15 and 1/20.

12. A unit dosage comprising the pharmaceutical composition according to any of the claim 7 - 11.

13. The unit dosage according to claim 12, wherein the unit dosage form comprises between 5 and 100 mg nefopam, preferably between 10 and 50 mg nefopam and even more preferred between 20 and 40 mg nefopam.

14. The unit dosage according to claim 12 or 13, wherein the unit dosage form comprises between 100 and 1000 mg acetaminophen, preferably between 200 and 750 mg acetaminophen and even more preferred between 300 and 600 mg acetaminophen.

15. The unit dosage according to claim 12, 13 or 14 wherein said unit dosage is a liquid unit dosage or a solid unit dosage, e.g. a tablet or a capsule.
